# EUROPEAN PATENT SPECIFICATION

(11) **EP 1 180 168 B1**
(45) Date of publication and mention of the grant of the patent: **17.11.2004**
(21) Application number: 00938685.5
(22) Date of filing: 26.05.2000
(51) Int. Cl.: C12Q 1/68, C07K 14/47, C07K 19/00, A61K 39/00, C12N 5/10, A61K 48/00, A01K 67/027

(54) **MOLECULAR CHARACTERISATION OF CHROMOSOME TRANSLOCATION t(11;18)(q21;q21) AND ITS CORRELATION TO CARCINOGENESIS**
MOLEKULARE CHARAKTERISIERUNG DER CHROMOSOMALEN TRANSLOKATION T(11;18)(Q21;Q21) UND IHRER ZUSAMMENHANG MIT KREBS
CARACTERISATION MOLECULAIRE D'UNE TRANSLOCATION DE CHROMOSOMES t(11;18)(q21;Q21) ET SA CORRELATION AVEC LA CARCINOGENESE

(30) Priority: 27.05.1999 EP 99201683
(43) Date of publication of application: 20.02.2002
(73) Proprietor: Vlaams Interuniversitair Instituut voor Biotechnologie vzw., 9052 Zwijnaarde (BE)
(72) Inventor: BAENS, Matthijs, B-3210 Lubbeek (BE); MARYNEN, Peter, B-3020 Herent (BE); DIERLAMM, Judith, D-22395 Hamburg (DE)
(74) Representative: Brants, Johan Philippe Emile
(86) International application number: PCT/EP2000/004796
(87) International publication number: WO 2000/073500

(56) References cited:
- EP-A- 0 189 628
- WO-A-91/00364
- WO-A-97/06182
- ROTHE M ET AL: "THE TNFR2-TRAF SIGNALING COMPLEX CONTAINS TWO NOVEL PROTEINS RELATED TO BACULOVIRAL INHIBITOR OF APOPTOSIS PROTEINS" CELL,US,CAMBRIDGE,MA, vol. 83, no. 7, 29 December 1995 (1995-12-29), pages 1243-1252, XP000607784 ISSN: 0092-8674 cited in the application
- AKAGI T ET AL: "Molecular cytogenetic delineation of the breakpoint at 18q21.1 in low-grade B-cell lymphoma of mucosa -associated lymphoid tissue." GENES, CHROMOSOMES AND CANCER, (1999 APR) 24 (4) 315-21., XP000876511
- OTT ET AL.: "The t(11;18)(q21;q21) chromosome translocation is a frequent and specific aberation in low grade but not high grade malignant non-Hodgkin's lymphomas of MALT type 1" CANCER RESEARCH, vol. 57, September 1997 (1997-09), pages 3944-3948, XP002129854 cited in the application
- DIERLAMM, J. (1) ET AL: "Molecular cytogenetic characterization of the chromosome 11 and 18 breakpoints in the t(11;18) (q21;q21) associated with malt lymphomas." BLOOD, (NOV. 15, 1998) VOL. 92, NO. 10 SUPPL. 1 PART 1-2, PP. 240A. MEETING INFO.: 40TH ANNUAL MEETING OF THE AMERICAN SOCIETY OF HEMATOLOGY MIAMI BEACH, FLORIDA, USA DECEMBER 4-8, 1998 THE AMERICAN SOCIETY OF HEAMATOLOGY., XP000876516
- DATABASE BIOSIS [Online] BIOSCIENCES INFORMATION SERVICE, PHILADELPHIA, PA, US; 1 June 1999 (1999-06-01) DIERLAMM JUDITH ET AL: "The apoptosis inhibitor gene API2 and a novel 18q gene, MLT, are recurrently rearranged in the t(11;18)(q21;q21) associated with mucosa-associated lymphoid tissue lymphomas." Database accession no. PREV199900310877 XP002149031 & BLOOD, vol. 93, no. 11, 1 June 1999 (1999-06-01), pages 3601-3609, ISSN: 0006-4971
- AKAGI ET AL.: "A novel gene, MALT1 at 18q21, is involved in t(11;18)(q21;q21) found in low-grade B-cell lymphoma of mucosa-associated lymphoid tissue" ONCOGENE 18:5785-5794(1999), 14 October 1999 (1999-10-14), XP000867839

## Description

The invention relates to the fields of medicine and diagnostics. More in particular the invention relates to medicine and diagnostics of tumours.

Recurrent translocations acquired in a process of transformation are well recognised in nodal B-cell lymphomas. These translocations characterise distinct subtypes of disease and involve genes controlling cell proliferation and apoptosis. *BCL2,* which suppresses apoptosis, was cloned from the t(14;18)(q21;q32) found in most cases of follicular B-cell lymphoma, whereas translocations involving the *BCL1*/*CyclinD1* gene on chromosome 11q13 are seen in nearly all cases of mantle cell lymphoma.¹

By contrast, the genetic mechanisms underlying the genesis and disease progression of extranodal marginal zone B-cell lymphomas of the mucosa-associated lymphoid tissue (MALT) type, a recently recognised distinct subtype of B-cell Non-Hodgkin's Lymphoma's (NHL), are not known.² MALT lymphomas account for five to ten percent of all NHLs and the vast majority of lymphomas arising at extranodal sites. They originate in a setting of chronic inflammation triggered by chronic infection or autoimmune disorders, such as Helicobacter pylori gastritis, Sjögren's syndrome, and Hashimoto's thyreoiditis.³ In vitro experiments have shown that H. pylori specific T-cells provide contact dependent help for the growth of the malignant B-cells of gastric MALT.⁴ The etiological link between low-grade gastric MALT lymphomas and H. pylori infection has also been demonstrated by the regression of some cases with antibiotic therapy.^{5.6} The preferential use of immunoglobulin variable region genes (V_{H}) associated with autoimmune disorders indicate that some MALT lymphomas may arise from autoreactive B-cells.^{7.8}
Since biopsies of these lymphomas are relatively rarely subjected to cytogenetic analysis and their in vitro proliferation is often poor, abnormal karyotypic data have been published for only 46 low-grade MALT lymphomas ⁹⁻¹⁷, 5 extranodal small lymphocytic lymphomas of probable marginal zone origin ¹⁸⁻²⁰, and 23 high-grade gastric MALT lymphomas ^{14,15}. Recurrent abnormalities in these cases include trisomies of chromosomes 3, 7, 12, and 18^{11,17,21}, the t(1;14)(p22;q32) which has been described in two cases ¹⁷ and the t(11;18)(q21;q21). The t(11;18)(q21:q21) has been detected in 15 out of the 51 low-grade lymphomas arising from various extranodal sites ^{9,12,14,18-20} but in none of the high-grade MALT lymphomas or any other subtype of NHL. In the largest cytogenetic series, this translocation has been found in 7 out of 13 cases of low-grade MALT lymphomas with an abnormal karyotype. ¹⁴ These data clearly indicate that the t(11;18) represents the most frequent structural abnormality in low-grade MALT lymphomas and seems to specifically characterise this disease entity. An attempt to delineate the breakpoint at 18q21.1 has been described by Akagi et al. (Genes, Chromosomes and Cancer, 24 (1999): 315-321). A detailed characterization, however, is urgently needed.
In one aspect the present invention discloses a detailed molecular genetic characterisation of the 11q21 and 18q21 breakpoint regions in MALT lymphomas characterised by the t(11;18)(q21;q21 ). The invention further teaches that the *API2* gene also known as *c-IAP2* ²², *HIAP1* ²³ and *MIHC* ²⁴, an inhibitor of apoptosis, and a novel gene on 18q21, named MLT, are rearranged in this translocation. The invention also discloses that truncation of the *API2* gene distal to its three BIR domains and fusion of this truncated gene with the carboxy-terminal region of *MLT* may lead to increased inhibition of apoptosis and thereby confer a survival benefit to MALT type B-cell lymphomas. In other words, the present invention discloses that, surprisingly, truncation of the API2 gene and fusion to the new MLT gene is crucial for the development of MALT type B-cell lymphomas.

In one aspect the invention teaches that the t(11;18)(q21;q21) associated with extranodal marginal zone B-cell lymphomas of the MALT type results in the expression of a chimeric transcript fusing *5'-APl2* on chromosome 11 to *3'-MLT on* chromosome 18. The occurrence of the t{11;18) translocation in not less than 17 out of 51 published cases of low-grade MALT lymphomas ^{9,12,14,18-20} including the two cases described in the present invention, along with its presence as the sole cytogenetic abnormality in 16 out of the 17 reported cases indicates that the t(11;18) represents one of the main recurrent, disease-specific translocations in NHL.
Several observations point to the *AP12-MLT* fusion as the oncogenic lesion underlying the t(11;18). The chimeric cDNA was cloned from two independent tumours. In one case the genomic breakpoints were also cloned and the structure of both genes and the localisation of the breakpoints is in agreement with the expression of the fusion transcript. The cryptic deletion of the 3' part of APl-2 in case 1 precludes the expression of a reciprocal *MLT - APl2* transcript in this case. As a result of the deletion the 5'-end of the *MLT* gene is fused to the 5'-end of the *MMP20* gene on the der(18). As both genes are present on opposite strands of the DNA no *MLT-MMP20* transcript is expressed. Furthermore FISH experiments with PACs for respectively *MLT, APl2* and *MMP20* clearly suggest that in case 2 a balanced translocation occurred not involving a break in the *MMP20* gene further arguing against any significance of the *MLT-MMP20* fusion.
*APl2* belongs to the family of inhibitor of apoptosis proteins (lAP), which play an evolutionary conserved role in regulating programmed cell death in diverse species (WO 97/06182 to Rothe and Goeddel). The *lAP* genes were first identified in baculoviruses in which they demonstrated an ability to suppress the host cell apoptotic response to viral infection.³³ Subsequently, five human IAP relatives have been described: *NIAP, APl1* (also known as *clAP1, HIAP2, MIHB), APl2 (clAP2, HIAP1, MIHC), XlAP - hlLP* and survivin.^{22-24,34-37} The common structural features of all lAP family members is a motif termed baculovirus lAP repeat (BIR) occurring in one to three copies, a caspase recruitment domain or CARD³⁰ located between the BIR domain(s) and a carboxy-terminal zinc binding RING finger domain³¹ that is present in all lAPs with the exception of *NIAP* and survivin. The human *APl9* and *APl2* proteins were originally identified as proteins that are recruited to the cytosolic domain of the tumour necrosis factor (TNF) receptor Il via their association with the TNF-associated factor (TRAP) proteins, TRAF-1 and TRAF-2 ²²and have been subsequently shown to suppress different apoptotic pathways by inhibiting distinct caspases, such as caspase-3, caspase-7, and pro-caspase-9.^{35,38}
The function of the novel *MLT* gene (also known as MALT1 ) located on chromosome 18q21 is not yet known. Its closest homologue is a hypothetical C. elegans gene. The carboxy-terminal part of this gene is characterised by the presence two lg-like C2-type domains and a domain similar to the murine lg gamma chain VDJ4 sequence (Accession no. M13070). The C2 domains are only present in the longer fusion cDNA of case 2 and thus probably have no functional significance in the tumour.
The molecular mechanism of action of the *APl2-MLT* fusion remains to be elucidated. Without being limited by theory it is hypothesised that the fusion protein resulting from the t(11;18) may lead to increased inhibition of apoptosis and thereby confer a survival advantage to MALT lymphomas and allow antigen-independent proliferation. Indeed, MALT lymphomas have been shown to display low levels of apoptosis³⁹, to escape from FAS-mediated apoptosis⁴⁰ and about 20% of low-grade MALT lymphomas do not respond to H. pylori eradication therapy.⁶ The truncation of *APl*2 after the BIR domains could release their anti-apototic effects from regulation by the CARD and RING domains. Recent studies have shown that the BIR domain-containing regions of *APl1* and *APl2* are sufficient for inhibition of caspases and suppression of apoptosis³⁵ The BIR domains of one of the *Drosophila* homologues (DlAP1 ) were demonstrated to suppress apoptosis in the *Drosophila* eye disk, whereas the full-length protein exhibited less activity. Moreover, transgenic flies overexpressing the RING domain alone exhibited increased cell death in the eye, suggesting that the RING domain may act as a negative regulator of cell death suppression in some instances.³⁴ On the other hand a specific role for the carboxy-terminal *MLT* domain is suggested by its consistent presence in the fusion and by the recurrency of the t(11;18) in MALT-lymphoma. This is supported by the observation that full-length *APl1* and *APl2* were somewhat more potent in caspase inactivation than constructs lacking the RING domain.³⁵ It is possible that the presence of the *MLT* domain would stabilise the fusion protein, increase its affinity for protein interaction or influence its subcellular localisation thereby modulating its interactions with other proteins.
The mechanism of gene deregulation by the t(11;18) differs from that seen in most of the B-cell lymphoma-associated translocations, which involve one of the immunoglobulin loci on 14q32, 2p12, or 22q11 and lead to deregulated expression of the incoming oncogene due to the proximity of potent B-cell transcriptional enhancers within the immunoglobulin loci.⁴¹ In this case the expression of the fusion gene is driven from the promoter of its 5' partner, *APl2.* The observation that APl2 mRNA is highly expressed in adult lymphoid tissues, including spleen, thymus, and peripheral blood lymphocytes and also in fetal lung and kidney is in agreement with this.²³. It is also interesting to note that the IAP family member survivin is strongly expressed in apoptosis-regulated human fetal tissues, but not in terminally differentiated adult tissues.⁴² Survivin becomes prominently expressed in transformed cell lines and in most human cancers. Survivin expression was also found in 50% of high-grade NHL (centroblastic, immunoblastic), but not in low-grade lymphomas (lymphocytic).³⁷

At the genomic level the rearrangements appear to be heterogeneous. The breakpoint in *MLT* occurred in two different introns for both cases. In the *APl2* gene the breakpoint occurred in the same intron for both cases but it was associated with the deletion of the 3'-end of the gene in only one tumour. The cytogenetic analysis of MALT lymphoma is often hampered by their poor proliferation *in vitro.* However, the physical maps and the genomic clones that the present invention teaches allows the development of a wide variety of sensitive detection methods for this rearrangement, such as but not limited to interphase FISH assays and assays based on the specific amplification of nucleic acid encompassing the t{11:18} breakpoint. Alternatively the fusion mRNA or the fusion protein provide new molecular targets for diagnosis.

A method for determining whether a tissue sample or an analogue and/or derivative thereof comprises a cell with a chromosome {11:18} translocation associated with malignancies such as mucosa-associated lymphoid tissue (MALT) lymphoma's is disclosed said method comprising subjecting nucleic acid from said sample to an amplification reaction using a primer that is complementary to a nucleic acid sequence which in humans lies on chromosome 11 region q21-22.3 and a primer that is complementary to a nucleic acid sequence which in humans lies on chromosome 18 region q21.1-22. and determining the presence of any amplified product. Preferably said tissue sample is taken from a human individual. Preferably said individual is suffering from or at risk of suffering from a disease.

Said nucleic acid may comprise chromosomal DNA, RNA or any other type of nucleic acid. With the knowledge of the region in which said {11:18} translocation occurs, a person skilled in the art is capable to develop specific nucleic acid amplification methods that allow the unambiguous detection of said translocation in a tissue sample. Such assays may be developed based on nucleic acid sequence information presented here. However, it is clear that using the identification of the break point and the methods and means to do so, additional nucleic acid sequence information on the region surrounding the break point can be obtained and that the use of such sequence information for the development of detection assays for said translocation falls within the scope of the present invention. With a complementary primer is meant a primer that can hybridise to another sequence under relatively mild hybridisation conditions, i.e. hybridisation conditions that allow nucleic acids with sequences with some mismatches to hybridise to each other. The number of mismatches among others determines the specificity and the efficiency of polymerisation. A complementary primer should comprise not more than 30% mismatches, preferably not more than 20% more preferably not more than 10%. Preferably said primer does not comprise mismatches.

Amplification methods that may be used include but are not limited to polymerase chain reaction, NASBA and intracellular PCR.

In a preferred embodiment said amplified product comprises a linked nucleic acid comprising at least part of nucleic acid encoding MALT-Lymphoma associated Translocation (MLT) protein and at least part of nucleic acid encoding apoptosis inhibitor 2 (APl2).

In a preferred embodiment said tissue sample comprises a lymphocyte, preferably an activated lymphocyte. In a preferred embodiment said tissue sample comprises digestive tract cells and/or stomach cells.

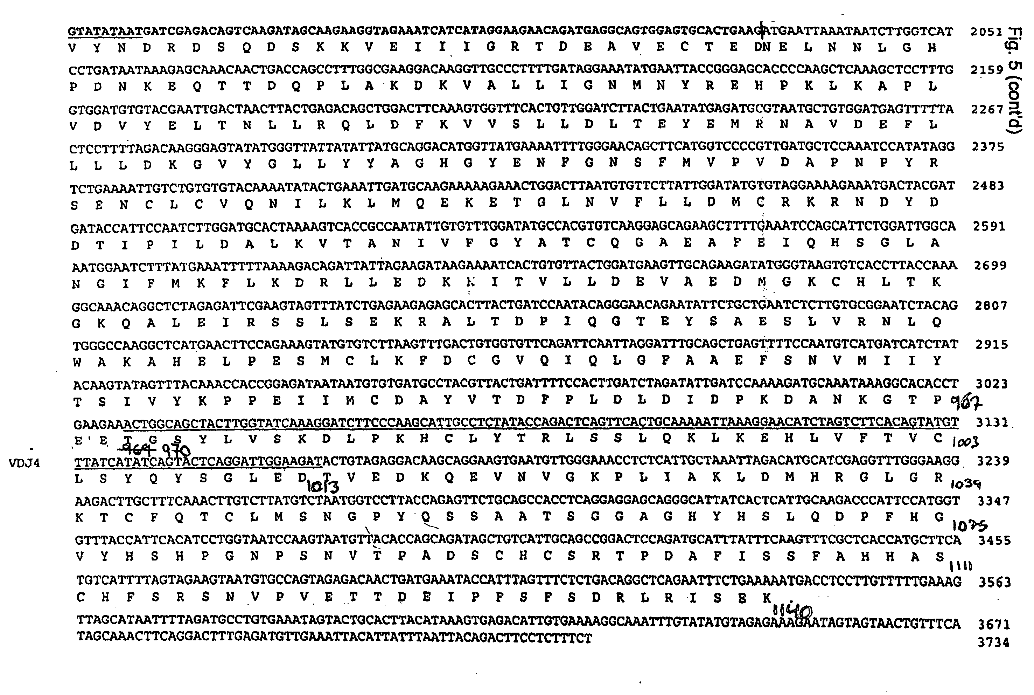

In another aspect the invention provides an isolated and/or recombinant nucleic acid encoding a proteinaceous molecule comprising at least the three BIR domains of apoptosis inhibitor 2 (API2) or a functional part, derivative and/or analogue thereof fused to at least the VDJ domain of MALT-Lymphoma associated Translocation (MLT) protein. Preferably said nucleic acid molecule comprises in humans a sequence as depicted in figure 5.

A nucleic acid of the invention may further comprise other nucleic acid. Other nucleic acid may facilitate cloning or amplification in bacteria. Preferably said other nucleic acid comprises nucleic acid corresponding in humans to chromosome 11 region q21-22.3. In this way said nucleic acid has advantageous properties for FISH analysis. Preferably said other nucleic acid comprises nucleic acid corresponding in humans to chromosome 18 region q21.1-22. In this way said nucleic acid has advantageous properties for FISH analysis.

The invention further provides a proteinaceous molecule encoded by a nucleic acid to the invention. Said protein may be advantageously used for the at least in part transforming a primary cell, preferably a B-cell and thus enabling and/or facilitating the generation of in vitro growing cell derivatives of said cell. One method of providing a cell with a proteinaceous molecule is through providing said cell with an expressible nucleic acid encoding said proteinaceous molecule and culturing said cell to obtain expression of said proteinaceous molecule. Preferably said proteinaceous molecule comprises a sequence which in humans is a sequences as depicted in Figure 5.

In another aspect the invention provides an isolated recombinant nucleic acid comprising the nucleic acid from position 2030 to 3542 of Fig. 5 encoding a MALT-Lymphoma associated Translocation (MLT) protein which in humans comprises a sequence as depicted in figure 5. The invention further provides a MALT-Lymphoma associated Translocation (MLT) protein encoded by a nucleic acid mentioned heretofore.

The invention further provides an antibody specific for a proteinaceous molecule according to the invention. It is clear to the person skilled in the art that antibodies can be generated in many ways once a suitable antigen has been identified. Antibodies can be generated through for instance the immunisation of an animal or human with a proteinaceous molecule of the invention. Alternatively, suitable peptides can be generated and used using standard protocols used in the art to generate antibodies in a mammal. However, antibodies can also be generated completely artificially from for instance libraries of synthetic antibodies, single chain antibodies, FAB fragment libraries etc. Suitable antibodies may be isolated from such libraries through techniques known in the art.

In another aspect the invention provides the use of a nucleic acid of the invention as a probe. With the term probe is meant a means for detection. A nucleic acid of the invention may be used as a probe, for instance but not limited to, for hybridisation to Southern Blot DNA of cells or for in situ hybridisation of cells, to determine the presence of complementary DNA to said nucleic acid. Information regarding the presence of such DNA may be used for determining the presence or absence of cells comprising said {11:18} translocation. Similarly, an antibody of the invention may be used for the determination of cells expressing a proteinaceous molecule of the invention.

In yet another aspect the invention provides a nucleic delivery vehicle comprising a nucleic acid according to the invention. Preferably said nucleic acid delivery vehicle comprises a virus particle, preferably an adenovirus particle, an adeno-associated virus particle and/or a retrovirus particle.

In yet another aspect the invention provides a cell comprising a nucleic acid according to the invention.

### EXAMPLES

### 1. Characterization of the 11q21 and 18q21 translocation and cloning of the fusion genes

### MATERIAL AND METHODS

### Tumour specimens

Two cases of low-grade extranodal gastrointestinal MALT lymphomas displaying the t(11;18)(q21;q21) were selected from the files of the Center for Human Genetics, University of Leuven, Belgium and the Department of Hematology, University of Salamanca, Spain based on the availability of metaphase spreads and frozen tumour tissue. Case 1 presented with an extended multifocal gastrointestinal MALT lymphoma involving the stomach, the small and large bowel, and the mesenteric lymphnodes. Case 2 was diagnosed with a gastric MALT lymphoma with secondary involvement of the spleen, the bone marrow, and the peripheral blood. Both cases revealed H. pylori associated gastritis and showed the typical morphology and immunophenotype of marginal zone B-cell lymphomas of MALT type including the characteristic tumour cell composition, extension of the marginal zones by tumour cells, follicular colonisation, lymphoepithelial lesions, expression of IgM, CD19, CD20, Ig_{κ} light chain restriction, and negativity for CD5, CD10, and CD23².

### Cytogenetic analysis

Cytogenetic analysis was performed as described" utilising tissue of a small bowel biopsy (case 1) and the spleen specimen (case 2). Both cases showed the t(11;18)(q21;q21 ) as the sole cytogenetic abnormality (case 1: 46,XY,t(11;18)(q21;q21) [17] /46,XY [3]; case 2: 46,XX.t(11;18)(q21;q21) [6]/46,X [14]). Fluorescence in situ hybridisation (FISH) was performed as previously described.²⁵ Chromosomes 11 and 18 were identified by cohybridisation with chromosome 11 (pLC11A) and 18 (L1.84) specific alpha-satellite probes in combination with G-banding using 4,6-diamidino-2-phenylindole-dihydrochlorid (DAPI) counterstain.

### Yeast artificial chromosome (YAC) clones

YAC clones derived from the Centre d'Etude du Polymorphisme Humain (CEPH) human megaYAC library were selected from the YAC contig reported by Chumakov et al.²⁶ and data obtained from URL http://www-genome.wi.mit.edu/cgi-bin/contig/yac_info at the Whitehead Institute for Biomedical Research. In addition, YAC A153A6 hybridising to the *BCL2* gene located at 18q21 ²⁷ and a probe specific for the *MLL* gene on 11q23 (Oncor, Gaithersburg, MD) were used. Human YAC inserts were selectively amplified using Alu-polymerase chain reaction (PCR).²⁸ In order to confirm their cytogenetic position and to determine the relative order of the YAC clones, pairs of differentially labelled YACs were hybridised to normal metaphase spreads obtained from PHA-stimulated peripheral blood lymphocytes of a healthy donor.

### P1 artificial chromosome (PAC) and plasmid clones

PAC clones were isolated by screening high-density filters from the RPCI libraries with ³²P-labeled probes. A walking strategy was used to extend the map. PAC end-fragments were rescued using a vectorette ligation approach.²⁹ The presence of the STS in the relevant PACs was confirmed by PCR and each PAC was analysed by FISH on normal metaphase spreads.
*BamHI* subclones of PAC 152M5 were generated by ligation of gel-purified fragments in pUC18 (Pharmacia Biotech, Uppsala, Sweden), and transformation into XL10-gold cells (Stratagene, La Jolla, CA). A *BamHI* restriction map was generated by comparing the sequence of the ends of the *BamHl* fragments to the sequence of random 1 kb subclones selected for containing the *BamHI* restriction sites. To generate random subclones of PAC 152M5, DNA was sheared by sonication, the fraction around 1 kb was gel-purified (Qiaquick Gel Extraction, Qiagen, ) blunted and ligated in pUC18, and transformed into XL1-blue cells.

### Reverse transcriptase (RT)-PCR and cloning

Total RNA was extracted from respectively tumour-infiltrated gastrointestinal and splenic tissue using the Trizol Reagent (Life Technologies, Inc., Rockville, Maryland). First strand cDNA was reverse transcribed from 1µg of total RNA with Murine Moloney Leukemia Virus reverse transcriptase (Life Technologies, Inc., Rockville, Maryland) according to standard procedures using a random hexamer primer. After size fractionation on Microspin S-400 HR columns (Pharmacia Biotech), a poly-A tail was added to the first strand cDNA with dATP and terminal deoxynucleotidyl transferase (Boehringer Mannheim, Mannheim, Germany). Double stranded cDNA was then generated using standard procedures with primer R2T8 (5' CCAGTGAGCAGAGTGACGAGGACTCGAGCTCAAGCTTTTTTTT 3'). Nested PCR was performed using respectively primers *MLTr1* (5' CCTTCTGCAACTTCATCCAG 3') and *MLTr2* (5' ATGGATTTGGAGCATCAACG 3') in combination with primers R2F1 (5' CCAGTGAGCAGAGTGACG 3') and R2F2 (5' GAGGACTCGAGCTCAAGC 3'). Amplification products were cloned in pGEM T-easy (Promega, Madison, WI).
The *AP12-MLT* fusion was confirmed by RT-PCR on patient RNA using the Titan RT-PCR system (Boehringer Mannheim) with primers *API2f1* (5' CCAAGTGGTTTCCAAGGTGT 3') and *MLTr2* and by sequence analysis of the cloned amplification products.
A partial *MLT* cDNA was isolated by 3' RACE on cDNA of patient 1. Several overlapping clones were analysed to obtain the 3' end sequences of *MLT* (Genbank accession no. AF 130356).

### RESULTS

### FISH characterisation of the 11q21 and 18q21 translocation

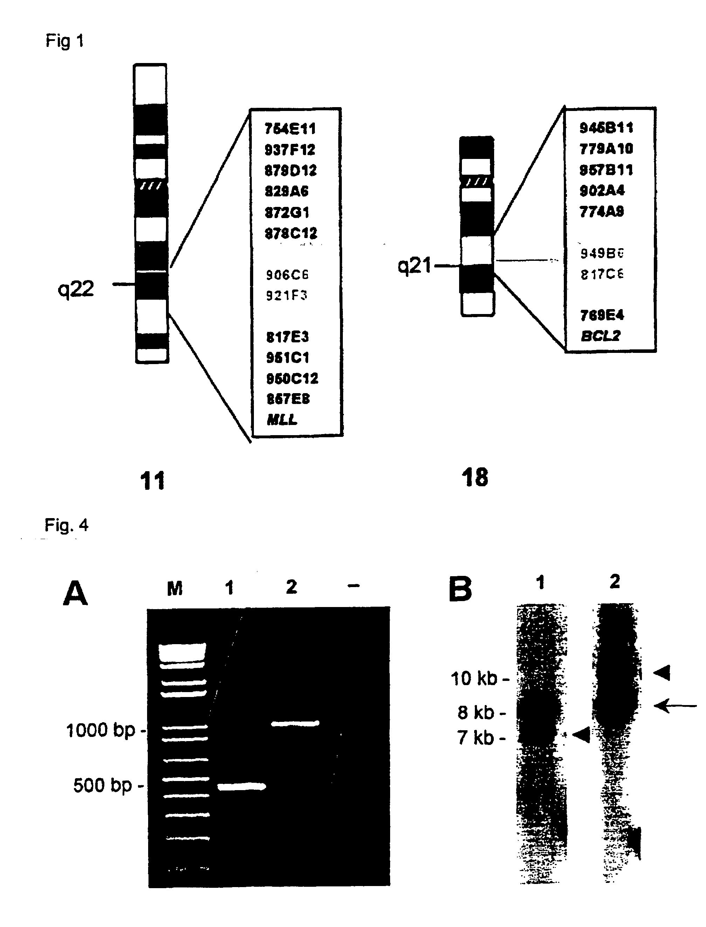

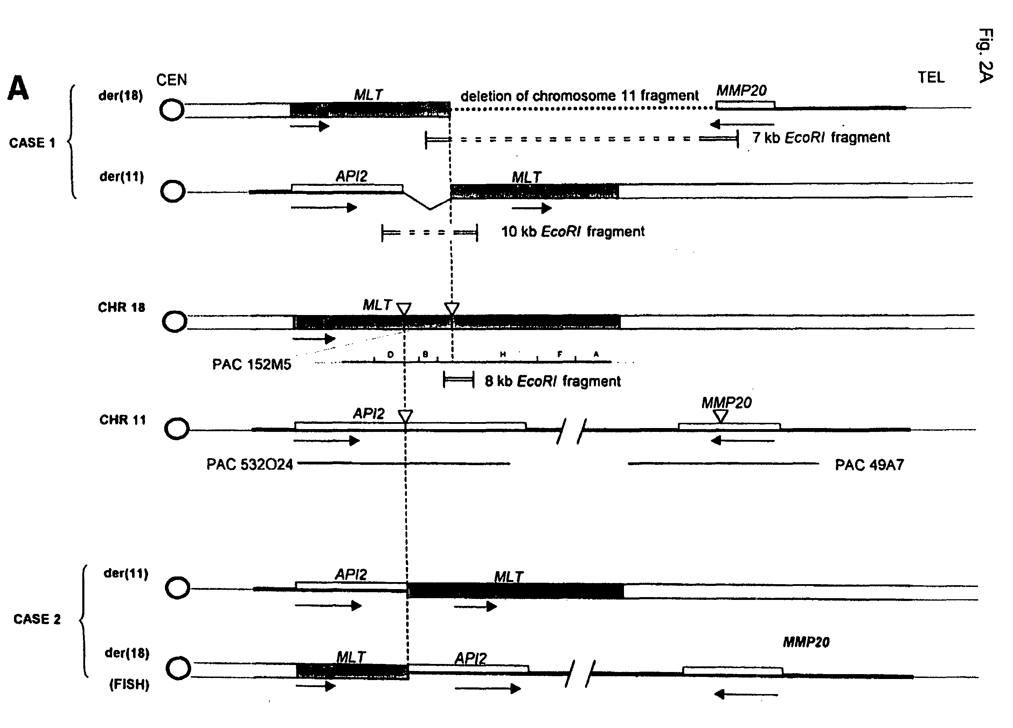

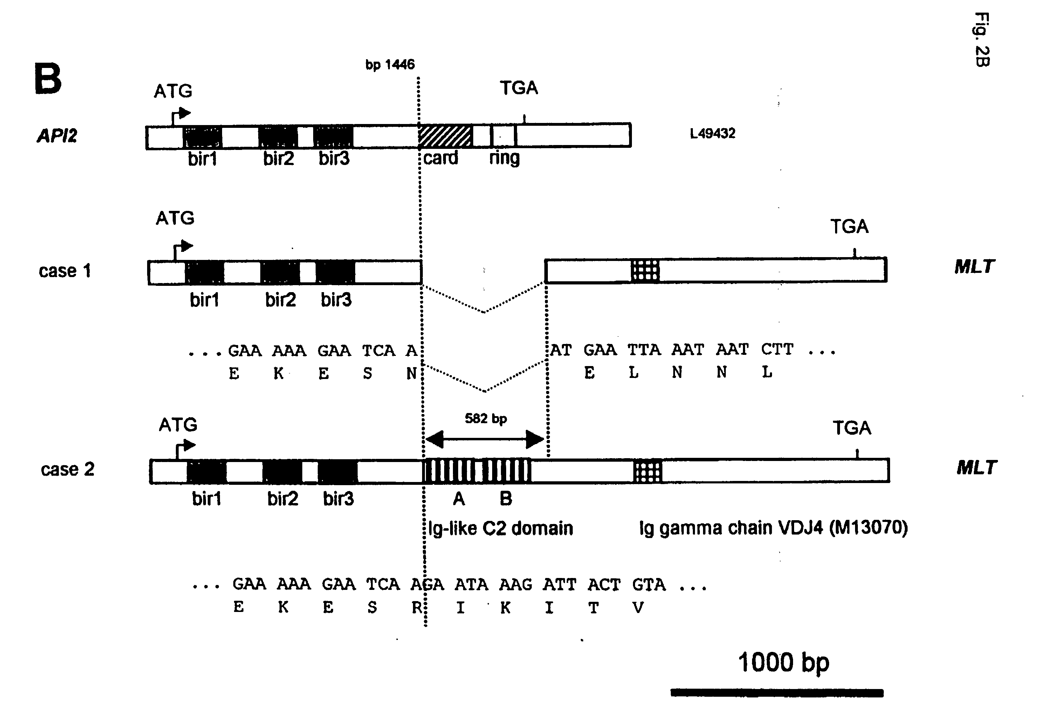

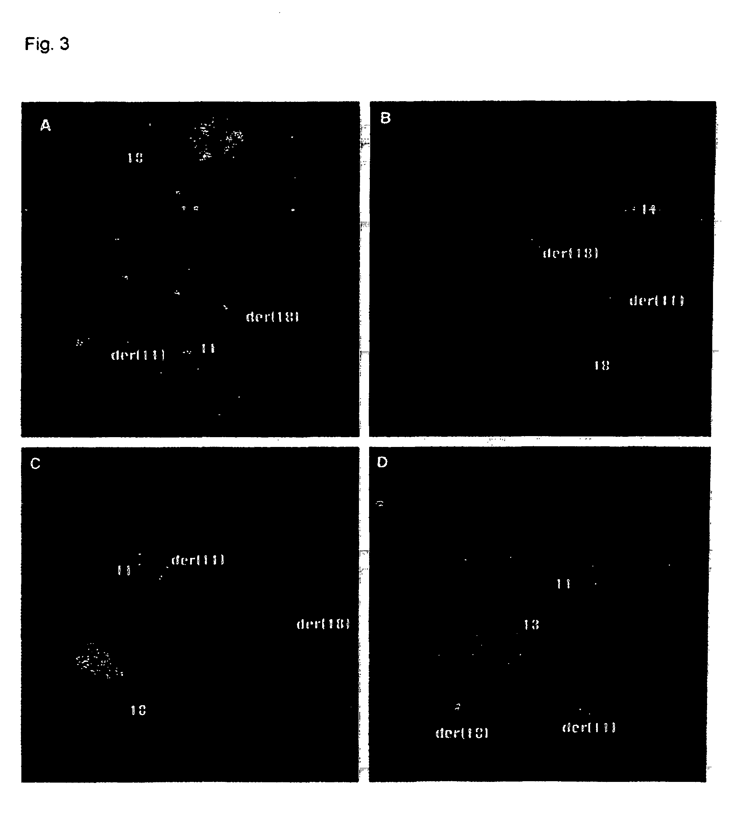

Twelve YACs derived from the chromosomal region 11q21-22.3 and the *MLL* probe were hybridised to metaphase spreads of case 1. Figure 1 shows their relative position in relation to the t(11;18) breakpoints. The hybridisation signals of YACs 906C5 and 921 F3 were split by the translocation. Subsequent analysis showed that YACs 906C5 and 921 F3 also spanned the translation breakpoint of case 2.
From the 9 YACs assigned to 18q21.1-22, five hybridised centromeric and two (including A153A6 containing the *BCL2* gene) telomeric to the translocation breakpoint (see fig. 1). The hybridisation signals of YACs 949B6 and 817C6 were split by the translocation in both cases.
YAC 949B6 contains 3 ordered STSs (cen - D18S887 - D18S1055 - D18S1129 - tel). FISH experiments with PAC clones isolated for these STSs positioned the chromosome 18 breakpoint in case 1 between D18S1055 and D18S1129. A walking strategy initiated from both markers led to the identification of PAC 205G9 and 152M5 which were shown to be split by the t(11;18) in both cases. The breakpoints were further narrowed down by FISH analysis with *BamHl* fragments subcloned from PAC 152M5 (Fig 2A). In case 1 fragment H was split by the translocation whereas in case 2 the breakpoint could be mapped to fragment D (Fig. 3).

### Cloning of the fusion genes

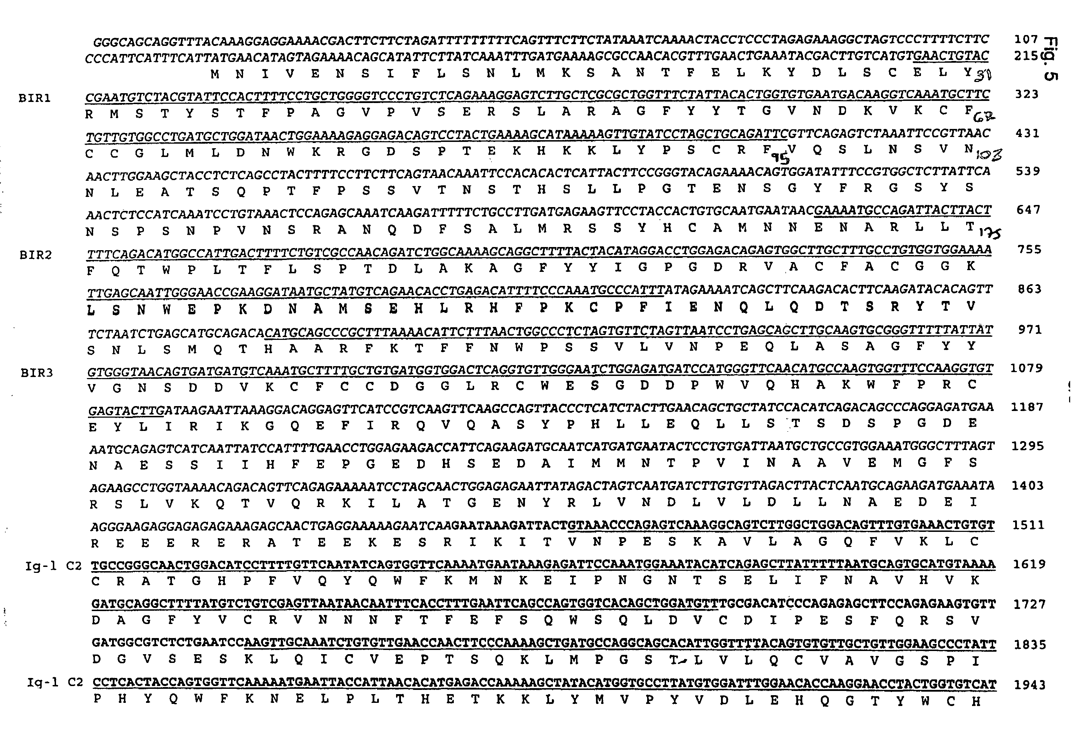

In order to identify genes on chromosome 18q in the vicinity of the breakpoints, the sequences derived from short random subclones from the BamHl fragments D, B, H and F were compared to the nucleotide databases. Subclone F24, mapping telomeric to the breakpoint, contained a 178 bp fragment identical to a single EST (IMAGE cDNA clone 1420842, GenBank accession no. AA826328) that resembles a hypothetical *Caenorhabditis elegans* gene (F22D3.6, Acc. No. U28993). The presence of canonical 5' and 3' splice sequences flanking the 178 bp suggested that this represented an exon of a human gene. A second exon with similarity to the same *C. elegans* gene product was predicted by computer analysis of subclone B9, located centromerically to the breakpoint in case 1. RT-PCR experiments confirmed that both exons were part of the same human transcript and indicated the disruption of this gene (named *MLT* for MALT-Lymphoma associated Translocation) by the translocation in case 1.
To identify an eventual chromosome 11 fusion partner for *MLT,* cDNA transcribed from RNA of case 1 was then used in 5' RACE experiments with two nested primers *(MLTr1* and r2) derived from *MLT* sequences telomeric to the breakpoint. The amplification products were cloned and eight clones with an average insert length of 800 bp were sequenced. Five clones contained uniquely *MLT* sequences. The three remaining clones showed a fusion of *MLT* sequences to the 5' part of the *APl2* gene, an inhibitor of apoptosis mapped to chromosome 11 q22. The *APl2* protein contains three copies of the baculovirus IAP repeat (BIR) at its amino-terminus and a caspase recruitment domain or CARD³⁰ followed by a carboxy-terminal zinc binding RING finger domain.³¹ The chimeric *API2 - MLT* transcript contains basepair 1 - 1446 of *APl2* (Genbank accession no. L49432) fused in frame with bp 583 of the partial *MLT* cDNA (Genbank accession no. AF130356). At the protein level the first 441 AA of *APl2,* containing the 3 BIR domains, are fused to the carboxy-terminal part of *MLT* (Fig. 2B).
Primers derived from the *APl2* and *MLT* cDNA sequences (M&M) were then used to confirm this fusion directly by RT-PCR. An amplification product with the expected size (445 bp) and sequence was obtained for patient 1 confirming the existence of the chimeric *APl2 -MLT* transcript. In contrast, using the same primers a 1000 bp RT-PCR product was obtained with the RNA sample of the second patient (Fig. 4 A). Sequence analysis of the cloned product again confirmed a *APl2 - MLT* fusion with a continuous open reading frame. The breakpoint in the APl2 gene occurred at the same position as described for patient 1. The chimeric cDNA however contained an additional 582 bp *MLT* sequence in agreement with the more centromeric localisation of the 18q breakpoint in this case as defined by FISH (Fig. 28).
To complete the sequence of the chimeric mRNA, 3'RACE experiments were performed. The consensus cDNA sequences for both *APl2*/*ML*T fusions are shown in figure 5.

### Absence of a reciprocal MLT-APl2 transcript

To analyse the genomic events leading to the expression of a chimeric *APl2 - MLT* transcript we cloned the genomic breakpoints of case 1. To this aim an 8kb *EcoRl* fragment spanning the breakpoint was subcloned from fragment H. Southern hybridisation with the 5'- and the 3'-endfragment of this clone detected rearranged EcoRl fragments of respectively 6kb containing the 5'-end of *MLT* and 10 kb containing the 3'-end of *MLT* (Fig. 4B). Long-distance inverse PCR³² was used to amplify the genomic chromosome 11 sequences present in both chimeric fragments and PAC clones corresponding to these chromosome 11 sequences were isolated. To our surprise two independent sets of PAC clones were obtained. PAC 532024 was isolated using chromosome 11 sequences derived from the der(11). This PAC was shown to contain the 5' end of *APl2.* FISH experiments with this PAC yielded signals on the normal 11 and the der(11) of case 1. PAC 49A7, obtained with chromosome 11 sequences derived from the der(18) however did not contain *APl2* and sequencing showed that this clone contained the *MMP20* gene instead (Fig 2B). FISH experiments with this clone on case 1 resulted in fluorescent signals on the normal 11 and the der(18). Taken together these data show that in case1 the t(11;18) is associated with a cryptic deletion of chromosome 11 sequences distal to the breakpoint resulting in the absence of a *MLT - APl2* fusion. These data are in agreement with a localisation of the MMP gene cluster telomeric to *APl2.* As the exact distance is not know we cannot estimate the size of the deletion. Sequencing of the der(18) fusion fragment showed that the *MLT* gene and the *MMP20* gene are on opposite strands of the genome thereby excluding the expression of a *MLT-MMP20* transcript. FISH experiments on case 2 detected a signal for the PAC 532024 on the normal 11, the der(11) and the der(18) consistent with the occurrence of a balanced translocation and a breakpoint in *APl2.* PAC 49A7 yielded fluorescent signals on the normal 11 and the der(18) consistent with the localisation of the *MMP20* gene distally to *APl2.*

### 2. Evaluation of the oncogenic properties of the AP12-MLT fusion

Two strategies are applied to evaluate the oncogenic properties of the APl2-MLT fusion in vivo.
In a first approach we generate via a bone-marrow transplant (BMT) mice that express the AP12-MLT fusion in their bone marrow cells. Lethally irradiated recipient mice are rescued by a transplantation with donor bone marrow earlier retrovirally infected with a construct that expresses the APl2-MLT fusion protein.
In a second approach the APl2-MLT fusion are introduced in a germline of mice through a human chromosomal vector (HCV). After incorporation of an APl2-MLT fusion construct in the HCV via Cre-mediated homologous recombination in hamster cells, the vector is shuttled via microcell-mediated chromosome transfer to mouse ES cells for the generation of transchromosomal (transgenic) mice. B-cell specific expression of the APl2-MLT fusion protein is achieved by using a B-cell specific promotor or by removal of a repressor element via a recombinase-mediated system driven by a B-cell specific promotor.
The mice models are used to assess the role of antigen triggering in the development and growth of gastric MALT lymphomas by infection with Helicobacter felis. Mice developing lymphoma provide the appropriate model to evaluate antibiotic therapies and therapies based on immune responses to the fusion protein.

### 3. Structure of the MLT Gene and Molecular Characterisation of the Genomic Breakpoint Junctions in the t(11;18)(q21;q21) of marginal zone B-cell Lymphomas of MALT type

The present example relates to the characterization of the genomic organization of the *MLT* gene. The information is used to amplify the genomic breakpoint junctions for five MALT-type lymphomas with t(11;18)(q21;q21). Sequences near the breakpoint junctions do not yield evidence for the participation of site-specific recombinases or *Alu*-mediated homologous recombination in the generation of the t(11;18). Clustering of the breakpoints in intron 7 of *API2* and the consistency of 'in-frame' *API2-MLT* fusions therefore point to a selective advantage associated with these fusions which leads to their clonal outgrowth.

### MATERIALS AND METHODS

### Patients

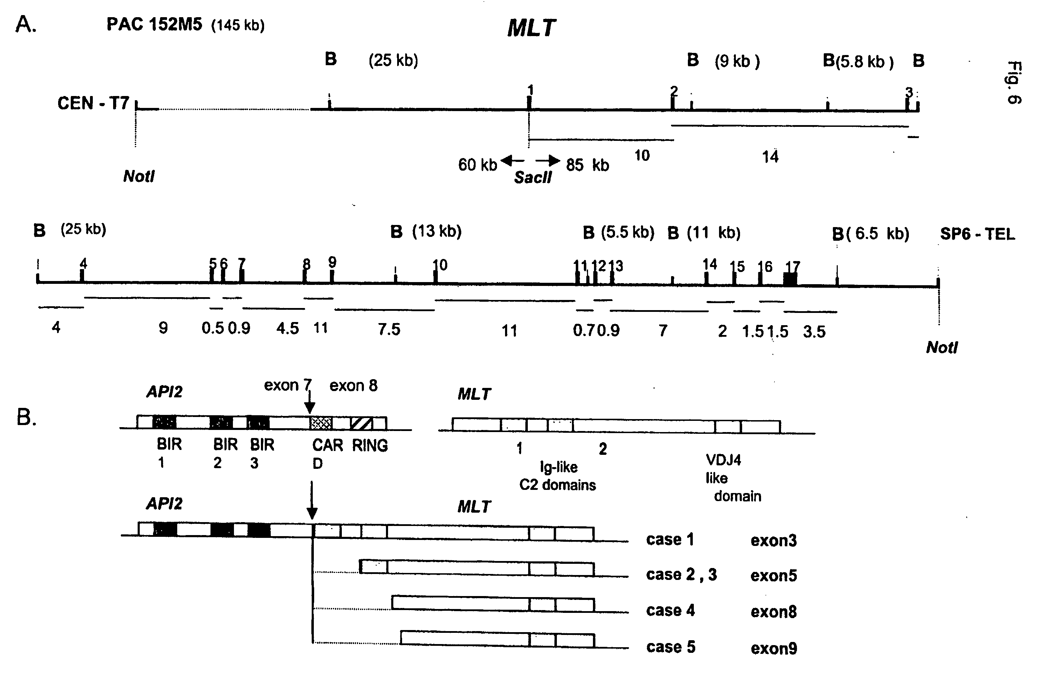

Five patients with gastric marginal zone B-cell lymphoma of MALT-type were investigated. The t(11;18) was confirmed by RT-PCR analysis for the *APl2-MLT* fusion transcript (Baens et al. 2000). Case 4 and 1 are case 1 and 2 respectively of a previous study (Dierlamm et al. 1999). The features of the *APl2-ML T* fusions for these five cases is depicted in figure 6B. Genomic DNA was extracted by using a standard isolation procedure (Sambrook et al. 1989) from 5 sections of 25 micron thick, taken from a frozen tissue block comprising the lymphoma.

### The genomic structure of MLT.

A fetal kidney cDNA library in λgt11 was screened with a 5' MLT probe to determine the full open reading frame of the MLT gene. MLT cDNAs for the entire open reading frame were used as probes on high-density filters containing 1 kb subclones of PAC 152M5. Purified PAC DNA was sonicated and the ends were blunted by successive treatment with respectively T4 DNA polymerase (AP Biotech, Uppsala, Sweden) and Klenow DNA polymerase (AP Biotech) in the presence of the 4 dNTPs. The fraction between 800 and 1500 bp was gel-purified (Qiaquick Gel Extraction, Qiagen, Hilden, Germany) and cloned into pUC18, linearised with Smal and dephosphorylated (AP Biotech). Subclones identified by the MLT cDNA probes were sequenced to determine the exon-intron junctions. A vectorette PCR approach (Riley et al. 1990) was applied to amplify exon-intron boundaries absent in the subclone library.
Nucleotide sequencing reactions were carried out by dideoxynucleotide chain-termination with FITC-dATP or fluorescently labeled primers and analyzed on an Automated Laser Fluorescence sequencer (AP Biotech). Sequences were aligned with the Seqman software (DNAstar Inc). Repeatmasker was used to identify repetitive elements (http://ftp.genome.washington.edu/cgi-bin/RepeatMasker).

### Characterization of the genomic AP12-MLT and MLT-APl2 fusion fragments.

Nested or heminested amplification was performed using the Expand Long Template PCR System according to the manufacturer's recommendations (Roche Diagnostics, Mannheim, Germany). Primers used for amplification are summarized in table 2. PCR amplification products were gel-purified (Qiaquick Gel Extraction, Qiagen), phosphorylated with T4 polynucleotide kinase (AP Biotech), sonicated and the fraction between 800 and 1500 bp was gel purified and cloned in pUC18/Smal/BAP (AP Biotech). Intron 7 of APl2 was amplified using primers APl2-1374f and APl2-1546r with as template DNA of PAC 523024, containing the APl2 gene. Subcloning and sequencing was performed as described above. The genomic APl2-MLT and MLT-MMP20 fusion fragments of case 4 were characterized in a previous study (Dierlamm et al. 1999).

### RESULTS

### The genomic structure of the MLT gene.

The present invention discloses a MLT consensus cDNA sequence of 2491 bp (Accession number AF130356). A fetal kidney cDNA library was screened with a 5' cDNA probe and two hybridizing clones were identified. Sequence analysis revealed additional 5' sequences with an 'in frame' ATG start codon at position 165 (Accession number AF130356). To determine the genomic organization of MLT, we generated a 1 kb subclone library of PAC 152M5 containing the MLT gene. Southern hybridizations confirmed the presence of 5' and 3' MLT sequences in this PAC clone. MLT cDNAs were used as probes on high-density filters containing the 1 kb subclones of PAC 152M5. Sequence analysis of hybridizing clones yielded the flanking intron sequences for all exons except exon 14 (3' acceptor of preceding intron missing). A vectorette PCR approach (Riley et al. 1990) was applied to characterize the missing boundary for exon 14. Basepairs 1-373 of the consensus cDNA sequence belong to exon 1 and show a high GC content (78 %) with 48 CpG dinucleotides and recognition sequences for several rare cutting restriction enzymes (SaclI, NarI, Nael).

In total 17 exons all flanked by canonical splice donor and acceptor sites were identified (Table 1). The size of the introns was estimated by long range PCR with exon specific primers and/or restriction mapping. It shows that the MLT gene is approximately 80 kb in size (Table 1 and Fig. 6A).

### The genomic breakpoint junctions for 5 MALT-type lymphomas with t(11;18).

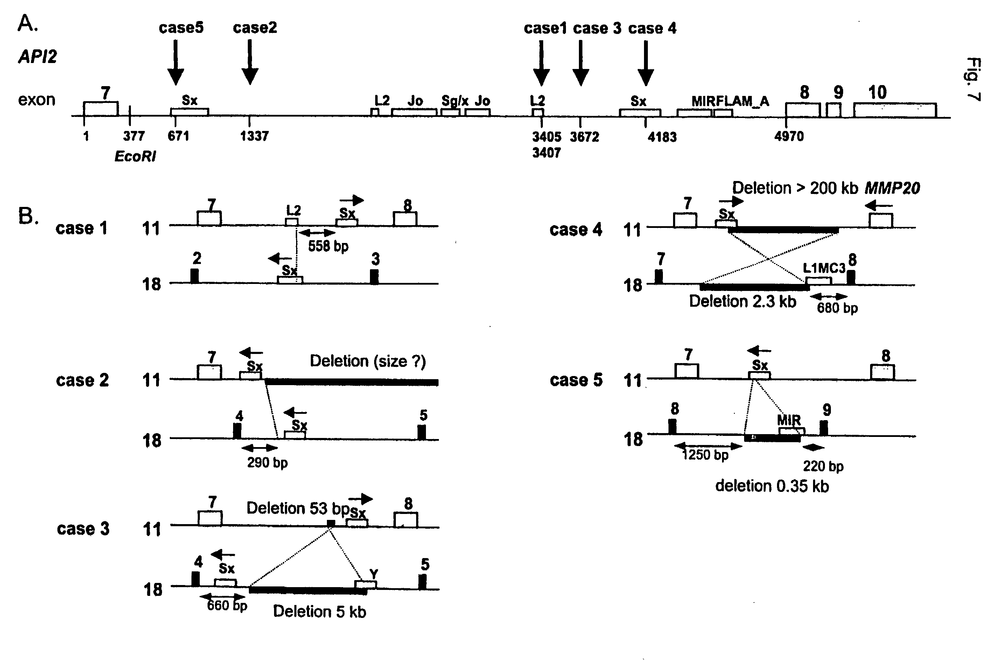

Five marginal zone B-cell lymphomas of MALT-type were selected from 11 cases identified with an APl2-MLT fusion as part of large screening of gastric lymphomas (Baens et al. 2000). All 11 cases had their chromosome 11 breakpoint in the 5 kb intron between exon 7 and 8 of APl2 (exon numbering according to Young et al. 1999). The genomic structure of MLT shows that the chromosome 18 breakpoint in these cases occurs upstream of exon 3, 5, 8 or 9 respectively and that the intron sizes permit amplification of the genomic fusion fragments using APl2 and MLT exon primers (Table 2). Genomic DNA extracted from a frozen tissue block comprising the lymphoma was used as template. Nested or heminested amplification yielded the genomic API2-MLT and MLT-API2 amplification products for all cases, except for case 2 where no MLT-API2 fragment could be amplified (Table 2). All fragments were partially sequenced until the breakpoint junctions were detected by comparison with the intron 7 sequence of API2 (Acc. No. AF178945). The sequence of the latter was determined from a 5 kb PCR fragment amplified from PAC 532024 with primers for exon 7 and 8 of API2 (Table 2). Sequences fused to API2 were then used to screen high-density filters containing the 1 kb subclones of PAC 152M5 to obtain the MLT sequences flanking the breakpoint.
The genomic breakpoints on chromosome 11 are scattered in intron 7 of API2 (Fig. 7A). Sequence analysis of the der(18) junction showed that the MLT gene is fused to MMP20 (Matrix MetalloProteinase 20). However both genes are transcribed in opposite orientations which excludes the expression of a MLT-MMP20 fusion transcript. Sequence analysis of the API2-MLT fusion further indicated a deletion (2.3 kb) of chromosome 18 sequences (Fig. 7B). Deletions of chromosome 18 sequences were also observed for case 5 (350 bp) and case 3 (around 5 kb), in the latter case simultaneously with a small deletion of chromosome 11 sequences (53 bp)(Fig. 7B). No MLT-APl2 amplification product was obtained for case 2. Based on the position of the der(11) breakpoint the expected fragment is at most 5 kb and thus well within amplification range, which suggests a deletion of chromosome 11 sequences including exon 8 of API2. The latter is supported by FISH experiments with PAC 166G16 for API2 which yielded hybridisation signals on the normal chromosome 11 and the der(11), but not the der(18). Identical FISH results were previously obtained for case 4 where the translocation was associated with a deletion of chromosome 11 sequences (> 200 kb), which explains the absence of hybridisation signals on the der(18) (Dierlamm et al. 1999). Only the translocation in case 1 is not associated with chromosomal loss (Fig. 7B).

### Characteristic sequences near the breakpoints.

Computer-based FINDPATTERN searches were performed to identify sequence motifs that might reveal a common mechanism for DNA breakage and rejoining. In lymphoid neoplasms, the presence of heptamer-nonamer recombination signals at or near the breakpoints suggests the involvement of V(D)J recombinase activity in the illegitimate recombination events leading to these translocations (Tycko and Sklar 1990). We did not find appropriate antigen receptor gene-like signals within the chromosome 11 and 18 sequences flanking the breakpoint junctions. The absence of non-template N nucleotides at the fusion junctions, characteristic for V(D)J recombination, further argues against its involvement.
Several other sequence motifs that could potentially infer recombination or genetic instability leading to chromosomal translocation have been reported, such as the DNA topoisomerase II binding and cleavage site (Negrini et al. 1993; Gu et al. 1994; Domer et al. 1995) χ-like sequences (Wyatt et al. 1992), the translin binding consensus sequence (Jaeger et al. 1993; Aoki et al. 1995) and alternating polypurine-polypyrimidine stretches (Boehm et al. 1989; Thandla et al. 1999; Wiemels and Greaves 1999; Thandla et al. 1999; Wiemels and Greaves 1999). None of these sites were consistently present near the breakpoint junctions. An 18/18 bp match for the topoisomerase II consensus sequence was only observed for case 1. Degenerated sequences (15 to 16 bp match on 18) were identified on one or both participating chromosomes for 4 out of 5 cases, but their position relative to the breakpoint does not imply any causality. Based on the same criteria a recombination event mediated by χ-like elements could be excluded. Furthermore no regions homologous to the Translin binding consensus sequence or with alternating polypurine/polypyrimidine stretches were apparent.
Homologous recombination between human Alu repeats located on the same or different chromosomes has been reported to cause chromosomal translocations (Jeffs et al. 1998; Strout et al. 1998). Alu repeats are also often observed in the vicinity of breakpoints and it is speculated that their mutual attraction juxtaposes these different chromosomal regions and in this way facilitates recurrent rearrangements between them (Obata et al. 1999). Repetitive sequences near the breakpoint junctions were identified using Repeatmasker and are summarised in Figure 7A and B. In two cases (4 and 5) the breakpoint in intron 7 of APl2 occurred in an AluSx repeat, although a different one, but no AluSx sequences were found close to the breakpoint site on chromosome 18. Case 1 had a breakpoint in a copy of an AluSx repeat on chromosome 18 but the break in intron 7 of APl2 was 558 bp upstream of the second AluSx repeat. For the remaining 2 cases (2, 3) AluSx repeats were detected close to the breakpoints on both chromosomes. For case 2 only the der(11) was characterised as no MLT-APl2 amplification product was obtained. The breakpoint was located 400 bp downstream an AluSx repeat on chromosome 11 and 52 bp upstream an AluSx repeat in intron 4 of MLT. The der(11) breakpoint for case 3 was located 291 bp upstream an AluSx repeat on chromosome 11 and 160 bp downstream an AluSx on chromosome 18. Due to deletions affecting both chromosomes the der(18) breakpoint was situated 238 bp upstream an AluSx on chromosome 11 and in an AluY repeat in intron 4 of the MLT gene (Fig. 7B).

### Brief description of the figures

### Figure 1: Cytogenetics and YAC characterisation of the t(11;18)

### Figure 2: Molecular structure of the t(11;18)

The genomic structure of the t(11 ;18) is shown in panel A. In the center the MLT gene is shown as the darkly shaded area on the normal 18q, *APl2* and *MMP20* are shown respectively as an open rectangle and a light grey rectangle on the normal 11q (not drawn to scale). Below each gene the PAC isolated for this gene is shown. For PAC 152M5 the position of the different *BamHl* fragments used for FISH experiments are indicated. On top the rearrangement in case **1** is illustrated: the der(11) fuses the 5' end of *APl2* to the 3' end of *MLT,* while on the der(18) as a result from the cryptic deletion of chromosome 11 the 5' end of *MLT* is fused to the 5' end of *MMP20.* The transcriptional orientation of each gene is indicated by an arrow below each chromosome, showing that on the der(18) MLT and MMP20 do have an opposite transcriptional orientation. The genomic fusion fragments which were cloned from respectively the der(11) and the der(18) are indicated by the double lines. Below the rearrangement of case 2 is shown: the der(11) fuses 5' *API2* to 3' *MLT.* The breakpoint in *API2* is identical to the one in case 1, the breakpoint in *MLT* occurred upstream of the breakpoint in case 1 (see 2B). FISH experiments suggest that the der(18) is the balanced reciprocal of the der(11). The localisations of all breakpoints are indicated on the normal chromosomes by open triangles.
Panel B illustrates the structure of the different fusion cDNAs. On top the structure of *API2* is shown with 3 aminoterminal BIR domains separated from the carboxyterminal RING domain by a CARD domain. The *API2* cDNA is truncated after the third BIR domain and fused in frame to *MLT.* As a result of the heterogeneity of the genomic breakpoints in case 2 582 additional nucleotides, encoding two Ig-like C2 domains of *MLT,* are present in this fusion. A Ig gamma VDJ4-like sequence in *MLT* is shown by a cross-hatched box. The sequence and the translation of the different junction fragments is shown underneath each cDNA.

### Figure 3: FISH Mapping of the chromosome 11 and 18 breakpoints

A: the hybridisation signals of YAC 921 F3 (green) and the *BamHI* fragment H of PAC 152M5 (red) are both split by the translocation in case 1. Signals of both probes are visible on the derivative chromosomes 11 and 18. B: in case 2, fragment D of PAC 152M5 (red) shows split signals. The centromeric probes for chromosome 11 and 18 appear in green. C: in case 1, PAC 532024 (green) is seen on the normal chromosome 11 and on the derivative chromosome 11 (C), whereas this probe is split by the translocation in case 2 (D). The signals of the centromeric probes are shown in red.

### Figure 4: Molecular characterisation of the fusions

(A) shows the *API2-MLT* products obtained by RT-PCR from case 1 and case 2. (B) shows the Southern blot detecting the rearranged *EcoRI* fragments of case 1. The probes were derived from an 8 kb *EcoRl* clone from chromosome 18 spanning the breakpoint (see fig 2A center). Lane 1 shows hybridisation with the probe derived proximally to the breakpoint, lane 2 shows hybridisation with the probe derived distally to the breakpoint. The arrow shows the normal 8kb EcoRI fragment, the arrowheads show the chimeric fragments

### Figure 5: Sequence of the APl2-MLT chimeric cDNA

**Figure 6: A.)** Genomic structure of the *MLT* gene. A (partial) *BamHl* (B) restriction map of PAC 152M5 is depicted. The sizes of ordered *BamHl* fragments are indicated, the last *BamHl* site of the first lane corresponds to the first one on the second lane. Solid rectangles represent the different MLT exons, their respective number is marked above. Solid lines delineate the *ML T* introns, their estimated size is specified underneath. The unique *Sacll* site is present in exon 1 of *MLT,* the size of the fragments obtained after *Notl* / *Sacll* digestion are indicated. B.) Features of *APl2, MLT* and observed *APl2-MLT* fusions of the five cases characterised are shown. *APl2* contains three amino-terminal BIR domains separated from the carboxy-terminal RING domain by a CARD domain. The arrow indicates the position of the *APl2* breakpoint observed in all cases, between exon 7 and exon 8. *MLT* harbours two Ig-like C2 domains and an Ig gamma VDJ4-like sequence.

**Figure 7: A)** The chromosome 11 breakpoints on the der(11) and sequence features of intron 7 of *APl2.* Numbering is according to Acc. No. AF178945. B) Schematic representation of the genomic structure of the *APl2 - MLT* and *MLT - APl2* fusion fragments in five cases (not drawn to scale). Grey and black boxes represent *APl2* and *MLT* exons respectively, white boxes repetitive elements. Solid black bars define deletions associated with the t(11;18). When known, the distance between the breakpoint and MLT exons is indicated. Abbreviations for repetitive elements (matching repeat # repeat class/family): AluSx # SINE/Alu (Sx), AluJo # SINE/Alu (Jo), AluSg/x # SINE/Alu (Sg/x), AluY # SINE/Alu (Y), L2 # LINE/L2, MIR # SINE/MIR, FLAM_A # SINE/Alu, L1 MC3 # LINE/L1.

### Tables

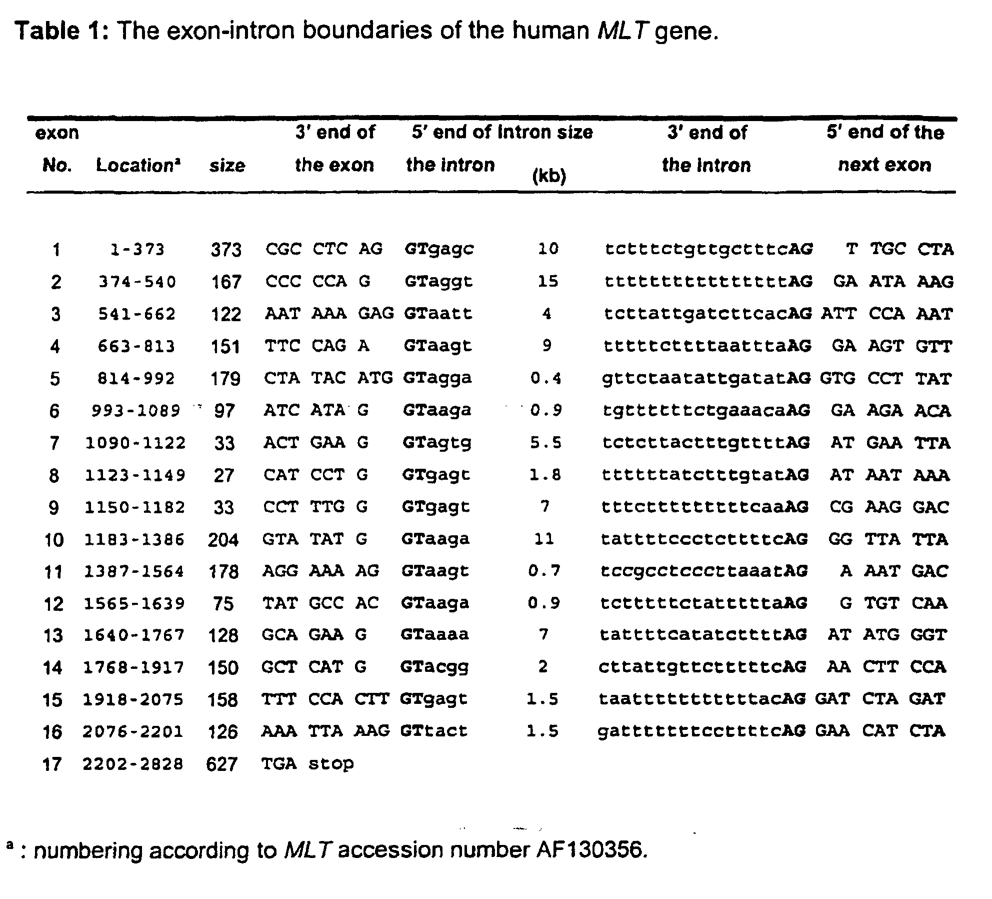

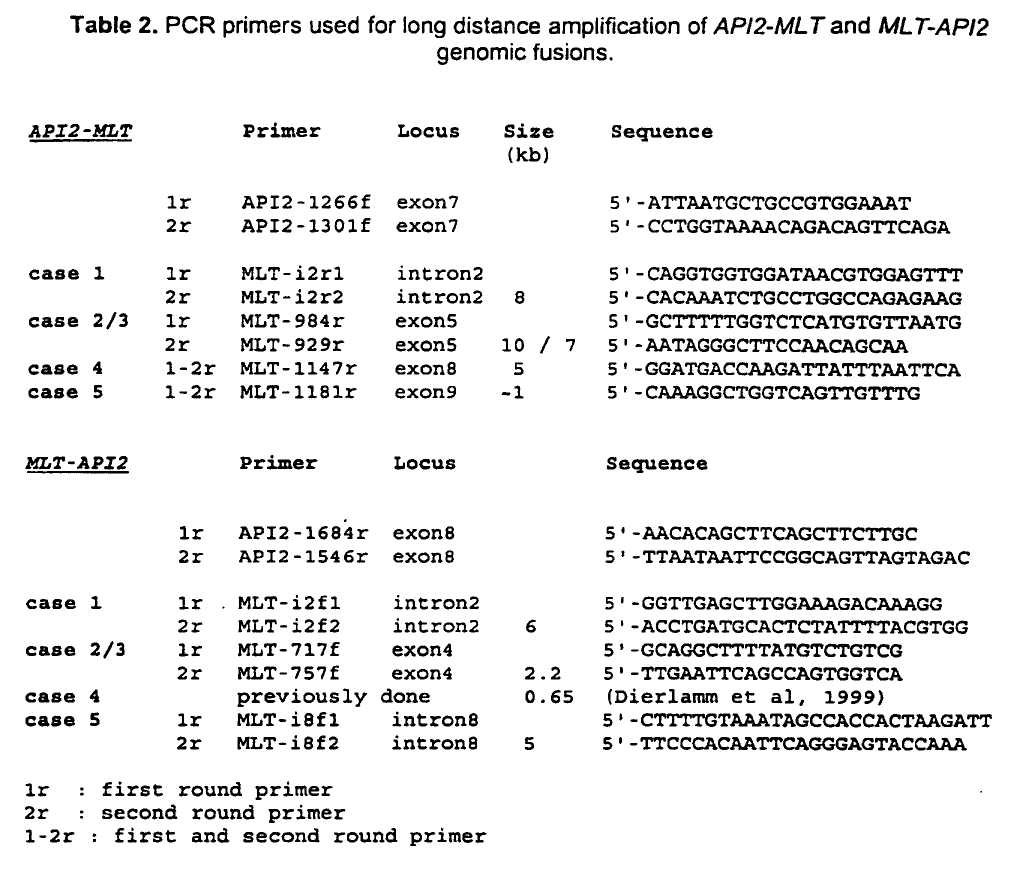

### Reference List

1. Tycko B, Sklar J: Chromosomal translocations in lymphoid neoplasia: a reappraisal of the recombinase model. Cancer Cells 2:1, 1990
2. Harris NL, Jaffe ES, Stein H, Banks PM, Chan JK, Cleary ML, Delsol G, De-Wolf-Peeters C, Falini B, Gatter KC: A revised European-American classification of lymphoid neoplasms: a proposal from the International Lymphoma Study Group [see comments]. Blood 84:1361, 1994
3. De-Wolf-Peeters C, Pittaluga S, Dierlamm J, Wlodarska I, Van-den-Berghe H: Marginal zone B-cell lymphomas including mucosa-associated lymphoid tissue type lymphoma (MALT), monocytoid B-cell lymphoma and splenic marginal zone cell lymphoma and their relation to the reactive marginal zone. Leuk.Lymphoma 26:467, 1997
4. Hussell T, Isaacson PG, Crabtree JE, Spencer J: Helicobacter pylori-specific tumour-infiltrating T cells provide contact dependent help for the growth of malignant B cells in low-grade gastric lymphoma of mucosa-associated lymphoid tissue [see comments]. J.Pathol. 178:122, 1996
5. Wotherspoon AC, Doglioni C, Diss TC, Pan L, Moschini A, de-Boni M, Isaacson PG: Regression of primary low-grade B-cell gastric lymphoma of mucosa-associated lymphoid tissue type after eradication of Helicobacter pylori [see comments]. Lancet 342:575, 1993
6. Bayerdorffer E, Neubauer A, Rudolph B, Thiede C, Lehn N, Eidt S, Stolte M: Regression of primary gastric lymphoma of mucosa-associated lymphoid tissue type after cure of Helicobacter pylori infection. MALT Lymphoma Study Group [see comments]. Lancet 345:1591, 1995
7. Qin Y, Greiner A, Trunk MJ, Schmausser B, Ott MM, Muller HH: Somatic hypermutation in low-grade mucosa-associated lymphoid tissue-type B-cell lymphoma. Blood 86:3528, 1995
8. Tierens A, Delabie J, Pittaluga S, Driessen A, DeWolf PC: Mutation analysis of the rearranged immunoglobulin heavy chain genes of marginal zone cell lymphomas indicates an origin from different marginal zone B lymphocyte subsets. Blood 91:2381, 1998
9. Auer lA, Gascoyne RD, Connors JM, Cotter FE, Greiner TC, Sanger WG, Horsman DE: t(11;18)(q21;q21) is the most common translocation in MALT lymphomas. Ann.Oncol. 8:979, 1997
10. Clark HM, Jones DB, Wright DH: Cytogenetic and molecular studies of t(14;18) and t(14;19) in nodal and extranodal B-cell lymphoma. J.Pathol. 166:129, 1992
11. Dierlamm J, Pittaluga S, Wlodarska I, Stul M, Thomas J, Boogaerts M, Michaux L, Driessen A, Mecucci C, Cassiman JJ, et a: Marginal zone B-cell lymphomas of different sites share similar cytogenetic and morphologic features [see comments]. Blood 87:299, 1996
12. Horsman D, Gascoyne R, Klasa R, Coupland R: t(11;18)(q21;q21.1 ): a recurring translocation in lymphomas of mucosa-associated lymphoid tissue (MALT)? Genes Chromosomes.Cancer 4:183, 1992
13. Kubonishi I, Sugito S, Kobayashi M, Asahi Y, Tsuchiya T, Yamashiro T, Miyoshi I: A unique chromosome translocation, t(11;12:18)(q13;q13;q12), in primary lung lymphoma. Cancer Genet.Cytogenet. 82:54, 1995
14. Ott G, Katzenberger T, Greiner A, Kalla J, Rosenwald A, Heinrich U, Ott MM, Muller HH: The t(11;18)(q21;q21) chromosome translocation is a frequent and specific aberration in low-grade but not high-grade malignant non-Hodgkin's lymphomas of the mucosa-associated lymphoid tissue (MALT-) type. Cancer Res. 57:3944, 1997
15. Robledo M, Benitez J, Rivas C, Martinez CP: Cytogenetic study of B-cell lymphoma of mucosa-associated lymphoid tissue [letter]. Cancer Genet.Cytogenet. 62:208, 1992
16. Whang PJ, Knutsen T, Jaffe E, Raffeld M, Zhao WP, Duffey P, Longo DL: Cytogenetic study of two cases with lymphoma of mucosa-associated lymphoid tissue. Cancer Genet.Cytogenet. 77:74, 1994
17. Wotherspoon AC, Pan LX, Diss TC, Isaacson PG: Cytogenetic study of B-cell lymphoma of mucosa-associated lymphoid tissue. Cancer Genet.Cytogenet. 58:35, 1992
18. Leroux D, Seite P, Hillion J, Le-Marc'hadour F, Pegourie BB, Jacob MC, Larsen CJ, Sotto JJ: t(11;18)(q21;q21) may delineate a spectrum of diffuse small B-cell lymphoma with extranodal involvement. Genes Chromosomes.Cancer 7:54, 1993
19. Levine EG, Arthur DC, Machnicki J, Frizzera G, Hurd D, Peterson B, Gajl PK, Bloomfield CD: Four new recurring translocations in non-Hodgkin lymphoma. Blood 74:1796, 1989
20. Griffin CA, Zehnbauer BA, Beschorner WE, Ambinder R, Mann R: t(11;18)(q21;q21) is a recurrent chromosome abnormality in small lymphocytic lymphoma. Genes Chromosomes.Cancer 4:153, 1992
21. Dierlamm J, Michaux L, Wlodarska I, Pittaluga S, Zeller W, Stul M, Criel A, Thomas J, Boogaerts M, Delaere P, Cassiman JJ, De-Wolf-Peeters C, Mecucci C, Van-den-Berghe H: Trisomy 3 in marginal zone B-cell lymphoma: a study based on cytogenetic analysis and fluorescence in situ hybridisation. Br.J.Haematol. 93:242, 1996
22. Rothe M, Pan MG, Henzel WJ, Ayres TM, Goeddel DV: The TNFR2-TRAF signaling complex contains two novel proteins related to baculoviral inhibitor of apoptosis proteins. Cell 83:1243, 1995
23. Liston P, Roy N, Tamai K, Lefebvre C, Baird S, Cherton HG, Farahani R, McLean M, Ikeda JE, MacKenzie A, Korneluk RG: Suppression of apoptosis in mammalian cells by NAIP and a related family of lAP genes. Nature 379:349, 1996
24. Uren AG, Pakusch M, Hawkins CJ, Puls KL, Vaux DL: Cloning and expression of apoptosis inhibitory protein homologs that function to inhibit apoptosis and/or bind tumour necrosis factor receptor-associated factors. Proc.Natl.Acad.Sci.U.S.A 93:4974, 1996
25. Dierlamm J, Wlodarska I, Michaux L, La Starza R: Successful Use of the Same Slide for Consecutive Fluorescence In Situ Hybridisation Experiments. Genes Chromosomes.Cancer 16:261, 1996
26. Chumakov IM, Rigault P, Le G, I, Bellanne CC, Billault A, Guillou S, Soularue P, Guasconi G, Poullier E, Gros I, Belova M, Sambucy J-L, Susini L, Gervy P, Gilbert F, Beaufils S, Bui H, Massart C, De Tand M-F, Dukasz F, Lecoulant S, OugenP, Perrot V, Saumier M, Soravito C, Bahouayila R, Cohen-Akenine A, Barillot E, Bertrand S, Codani J-J, Caterina D, Georges I, Lacroix B, Lucotte G, Sahbatou M, Schmidt C, Sangouard M, Tubacher E, Dib C, Fauré S, Fizames C, Gyapay G, Millaseau P, Nguyen S, Muselet D, Vignal A, Morissette J, Menninger J, Lieman J, Desai T, Banks A, Bray-Ward P, Ward D, Hudson TJ, Gerety SS, Foote S, Stein L, Page DC, Lander ES, Weissenbach J, Le Paslier D, Cohen D: A YAC contig map of the human genome. Nature 377:175,1995
27. Poetsch M, Weber MK, Plendl HJ, Grote W, Schlegelberger B: Detection of the t(14;18) chromosomal translocation by interphase cytogenetics with yeast-artificial-chromosome probes in follicular lymphoma and nonneoplastic lymphoproliferation. J.Clin.Oncol. 14:963, 1996
28. Lengauer C, Green ED, Cremer T: Fluorescence in situ hybridisation of YAC clones after Alu-PCR amplification. Genomics 13:826, 1992
29. Riley J, Butler R, Ogilvie D, Finniear R, Jenner D, Powell S, Anand R, Smith JC, Markham AF: A novel, rapid method for the isolation of terminal sequences from yeast artificial chromosome (YAC) clones. Nucleic Acids Res. 18:2887, 1990
30. Hofmann K, Bucher P, Tschopp J: The CARD domain: a new apoptotic signalling motif. Trends Biochem.Sci. 22:155, 1997
31. Saurin AJ, Borden KL, Boddy MN, Freemont PS: Does this have a familiar RING? Trends Biochem.Sci. 21:208,1996
32. Willis TG, Jadayel DM, Coignet LJ, Abdul RM, Treleaven JG, Catovsky D, Dyer MJ: Rapid molecular cloning of rearrangements of the IGHJ locus using long-distance inverse polymerase chain reaction. Blood 90:2456, 1997
33. Clem RJ, Fechheimer M, Miller LK: Prevention of apoptosis by a baculovirus gene during infection of insect cells. Science 254:1388, 1991
34. Hay BA, Wassarman DA, Rubin GM: Drosophila homologs of baculovirus inhibitor of apoptosis proteins function to block cell death. Cell 83:1253, 1995
35. Roy N, Deveraux QL, Takahashi R, Salvesen GS, Reed JC: The c-IAP-1 and c-IAP-2 proteins are direct inhibitors of specific caspases. EMBO J. 16:6914, 1997
36. Duckett CS, Nava VE, Gedrich RW, Clem RJ, Van-Dongen JL, Gilfillan MC, Shiels H, Hardwick JM, Thompson CB: A conserved family of cellular genes related to the baculovirus iap gene and encoding apoptosis inhibitors. EMBO J. 15:2685, 1996
37. Ambrosini G, Adida C, Altieri DC: A novel anti-apoptosis gene, survivin, expressed in cancer and lymphoma. Nat.Med. 3:917, 1997
38. Deveraux QL, Roy N, Stennicke HR, Van-Arsdale T, Zhou Q, Srinivasula SM, Alnemri ES, Salvesen GS, Reed JC: IAPs block apoptotic events induced by caspase-8 and cytochrome c by direct inhibition of distinct caspases. EMBO J. 17:2215, 1998
39. Du M, Singh N, Husseuin A, Isaacson PG, Pan L: Positive correlation between apoptotic and proliferative indices in gastrointestinal lymphomas of mucosa-associated lymphoid tissue (MALT). J.Pathol. 178:379, 1996
40. Greiner A, Seeberger H, Knörr C, Starostik P, Müfler-Hermelinck HK: MALT-type B-cell lymphomas escape the censoring FAS-mediated apoptosis. Blood 92 Suppl.1:#1997, 1998 (abstr.)
41. Jain VK, Judde JG, Max EE, Magrath IT: Variable IgH chain enhancer activity in Burkitt's lymphomas suggests an additional, direct mechanism of c-myc deregulation. J.Immunol. 150:5418, 1993
42. Adida C, Crotty PL, McGrath J, Berrebi D, Diebold J, Altieri DC: Developmentally regulated expression of the novel cancer anti-apoptosis gene survivin in human and mouse differentiation. Am.J.Pathol. 152:43, 1998
   - Akagi T, Motegi M, Tamura A, Suzuki R, Hosokawa Y, Suzuki H, Ota H, Nakamura S, Morishima Y, Taniwaki M, Seto M 1999 A novel gene, *MALT1* at 18q21, is involved in t(11;18) (q21;q21) found in low-grade B-cell lymphoma of mucosa-associated lymphoid tissue. Oncogene 18: 5785-5794.
   - Aoki K, Suzuki K, Sugano T, Tasaka T, Nakahara K, Kuge O, Omori A, Kasai M 1995 A novel gene, Translin, encodes a recombination hotspot binding protein associated with chromosomal translocations. Nat.Genet. 10: 167-174.
   - Aplan PD, Raimondi SC, Kirsch IR 1992 Disruption of the SCL gene by a t(1;3) translocation in a patient with T cell acute lymphoblastic leukemia. J.Exp.Med. 176: 1303-1310.
   - Baens M, Maes B, Steyls A, Geboes K, Marynen P, De Wolf-Peeters C. The product of the t(11;18), an *APl2-MLT* fusion, marks nearly half of the gastrointestinal MALT type lymphomas without large cell proliferation. Am. J. Pathol.156 : 1433-1439.
   - Baguley BC, Ferguson LR 1998 Mutagenic properties of topoisomerase-targeted drugs. Biochim.Biophys.Acta 1400: 213-222.
   - Bernard OA, Berger R 1995 Molecular basis of 11 q23 rearrangements in hematopoietic malignant proliferations. Genes Chromosomes.Cancer 13: 75-85.
   - Boehm T, Mengle-Gaw L, Kees UR, Spurr N, Lavenir I, Forster A, Rabbitts TH 1989 Alternating purine-pyrimidine tracts may promote chromosomal translocations seen in a variety of human lymphoid tumours. EMBO J. 8: 2621-2631.
   - Chissoe SL, Bodenteich A, Wang YF, Wang YP, Burian D, Clifton SW, Crabtree J, Freeman A, lyer K, Jian L, et a 1995 Sequence and analysis of the human ABL gene, the BCR gene, and regions involved in the Philadelphia chromosomal translocation. Genomics 27: 67-82.
   - Dierlamm J, Baens M, Wlodarska I, Stefanova OM, Hernandez JM, Hossfeld DK, De-Wolf-Peeters C, Hagemeijer A, Van-den-Berghe H, Marynen P 1999 The apoptosis inhibitor gene API2 and a novel 18q gene, MLT, are recurrently rearranged in the t(11;18)(q21;q21)p6ssociated with mucosa-associated lymphoid tissue lymphomas. Blood 93: 3601-3609.
   - Domer PH, Head DR, Renganathan N, Raimondi SC, Yang E, Atlas M 1995 Molecular analysis of 13 cases of MLL/11q23 secondary acute leukemia and identification of topoisomerase II consensus-binding sequences near the chromosomal breakpoint of a secondary leukemia with the t(4;11). Leukemia 9: 1305-1312.
   - Golub TR, Barker GF, Bohlander SK, Hiebert SW, Ward DC, Bray-Ward P, Morgan E, Raimondi SC, Rowley JD, Gilliland DG 1995 Fusion of the TEL gene on 12p13 to the AML1 1 gene on 21q22 in acute lymphoblastic leukemia. Proc.Natl.Acad.Sci.U.S.A 92: 4917-4921.
   - Gu Y, Alder H, Nakamura T, Schichman SA, Prasad R, Canaani O, Saito H, Croce CM, Canaani E 1994 Sequence analysis of the breakpoint cluster region in the ALL-1 gene involved in acute leukemia. Cancer Res 54: 2326-2330.
   - Harris NL, Jaffe ES, Diebold J, Flandrin G, Muller-Hermelink HK, Vardiman J, Lister TA, Bloomfield CD 1999 World Health Organization Classification of Neoplastic Diseases of the Hematopoietic and Lymphoid Tissues: Report of the Clinical Advisory Committee Meeting-Airlie House, Virginia, November 1997. J.Clin.Oncol. 17: 3835-3849.
   - Jaeger U, Purtscher B, Karth GD, Knapp S, Mannhalter C, Lechner K 1993 Mechanism of the chromosomal translocation t(14;18) in lymphoma: detection of a 45-Kd breakpoint binding protein. Blood 81: 1833-1840.
   - Jeffs AR, Benjes SM, Smith TL, Sowerby SJ, Morris CM 1998 The BCR gene recombines preferentially with Alu elements in complex BCR-ABL translocations of chronic myeloid leukaemia . Hum.Mol.Genet 7: 767-776.
   - Lochner K, Siegler G, Fuhrer M, Greil J, Beck JD, Fey GH, Marschalek R 1996 A specific deletion in the breakpoint cluster region of the ALL-1 gene is associated with acute lymphoblastic T-cell leukemias. Cancer Res 56: 2171-2177.
   - Maes B, Baens M, Marynen P, De Wolf-Peeters C. The product of the t(11;18), an API2-MLT fusion, is an almost exclusive finding in marginal zone cell lymphoma of extranodal MALT-type. Ann. Oncol., in press.
   - Maraschin J, Dutrillaux B, Aurias A 1990 Chromosome aberrations induced by etoposide (VP-16) are not random. Int.J.Cancer 46: 808-812.
   - Mitelman F, Mertens F, Johansson B 1997 A breakpoint map of recurrent chromosomal rearrangements in human neoplasia [see comments]. Nat.Genet. 15 Spec No: 417-474.
   - Negrini M, Felix CA, Martin C, Lange BJ, Nakamura T, Canaani E, Croce CM 1993 Potential topoisomerase II DNA-binding sites at the breakpoints of a t(9;11) chromosome translocation in acute myeloid leukemia. Cancer Res 53: 4489-4492.
   - Obata K, Hiraga H, Nojima T, Yoshida MC, Abe S 1999 Molecular characterization of the genomic breakpoint junction in a t(11;22) translocation in Ewing sarcoma. Genes Chromosomes.Cancer 25: 6-15.
   - Reichet M, Gillert E, Nilson I, Siegler G, Greil J, Fey GH, Marschalek R 1998 Fine structure of translocation breakpoints in leukemic blasts with chromosomal translocation t(4;11): the DNA damage-repair model of translocation. Oncogene 17: 3035-3044.
   - Romana SP, Poirel H, Leconiat M, Flexor MA, Mauchauffe M, Jonveaux P, Macintyre EA, Berger R, Bernard OA 1995 High frequency of t(12;21) in childhood B-lineage acute lymphoblastic leukemia. Blood 86: 4263-4269.
   - Rosenwald A, Ott G, Stilgenbauer S, Kalla J, Bredt M, Katzenberger T, Greiner A, Ott MM, Gawin B, hner H, IIer-Hermelink HK 1999 Exclusive Detection of the t(11;18)(q21;q21) in Extranodal Marginal Zone B Cell Lymphomas (MZBL) of MALT Type in Contrast to other MZBL and Extranodal Large B Cell Lymphomas. Am.J.Pathol. 155: 1817-1821.
   - Sambrook J, Fritsch E, Maniatis T 1989 Molecular cloning: A laboratory manual. Cold Spring Harbor, NY: Cold Spring Harbor Laboratory
   - Showe LC, Croce CM 1987 The role of chromosomal translocations. Annu.Rev.lmmunol. 5: 253-277.
   - Strout MP, Marcucci G, Bloomfield CD, Caligiuri MA 1998 The partial tandem duplication of ALL1 (MLL) is consistently generated by Alu-mediated homologous recombination in acute myeloid leukemia. Proc.Natl.Acad.Sci.U.S.A 95: 2390-2395.
   - Thandla SP, Ploski JE, Raza ES, Chhalliyil PP, Block AW, de-Jong PJ, Aplan PD 1999 ETV6-AML1 translocation breakpoints cluster near a purine/pyrimidine repeat region in the ETV6 gene. Blood 93: 293-299.
   - Toth G, Jurka J 1994 Repetitive DNA in and around translocation breakpoints of the Philadelphia chromosome. Gene 140: 285-288.
   - Tsujimoto Y, Gorham J, Cossman J, Jaffe E, Croce CM 1985 The t(14;18) chromosome translocations involved in B-cell neoplasms result from mistakes in VDJ joining. Science 229: 1390-1393.
   - Wiemels JL, Greaves M 1999 Structure and possible mechanisms of *TEL-AML1* gene fusions in childhood acute lymphoblastic leukemia. Cancer Res. 59: 4075-4082.
   - Wyatt RT, Rudders RA, Zelenetz A, Delellis RA, Krontiris TG 1992 BCL2 oncogene translocation is mediated by a chi-like consensus. J.Exp.Med. 175: 1575-1588.
   - Young SS, Liston P, Xuan JY, McRoberts C, Lefebvre CA, Korneluk RG 1999 Genomic organization and physical map of the human inhibitors of apoptosis: HIAP1 and HIAP2. Mamm.Genome 10: 44-48.

## Claims

1. An isolated and/or recombinant nucleic add encoding a proteinaceous molecule comprising at least the three BIR domains of apoptosis inhibitor 2 (APl2) fused to at least the VDJ4 domain of the MALT-lymphoma associated Translocation (MLT) protein having the sequence of amino acids 970-1013 of Figure 5.

2. An isolated and/or recombinant nucleic acid according to claim 1 wherein the nucleic acid sequence has Figure 5.

3. A nucleic delivery vehicle comprising a nucleic add according to claim 1 or 2.

4. A cell comprising a nucleic acid according to claim 1 or 2.

5. A proteinaceous molecule encoded by a nucleic acid according to claim 1 or 2.

6. A proteinaceous molecule according to claim 5 wherein the amino acid sequence has the sequence of Figure 5.

7. An isolated protein encoded by a nucleic acid comprising the nucleic acid from position 2029 position 3542 of the nucleic acid sequence as depicted in Figure 5.

8. Use of nucleic acid according to claim 1 or 2, a proteinaceous molecule according to claim 5 or 6, or a protein according to claim 7 or an antibody specific for a proteinaceous molecule according to claim 5 or 6 for in vitro diagnosis of mucosa-associated lymphoid tissue lymphoma's.

9. An antibody specific for a protein according to claim 7.

## Patentansprüche

1. Isolierte und/oder rekombinante Nukleinsäure, die für ein proteinöses Molekül kodiert, das wenigstens die drei BIR-Domänen des Apoptoseinhibitors 2 (API2), die an wenigstens die VDJ4-Domäne des MALT-Lymphom-assoziierten Translokation (MLT) Proteins fusioniert ist, umfasst, und das die Sequenz der Aminosäuren 970-1013 der Figur 5 hat.

2. Isolierte und/oder rekombinante Nukleinsäure gemäß Anspruch 1, wobei die Nukleinsäuresequenz die Sequenz aus Figur 5 hat.

3. Nukleinsäure-Zufuhrvehikel, umfassend eine Nukleinsäure gemäß Anspruch 1 oder 2.

4. Zelle, umfassend eine Nukleinsäure gemäß Anspruch 1 oder 2.

5. Proteinöses Molekül, das von einer Nukleinsäure gemäß Anspruch 1 oder 2 kodiert wird.

6. Proteinöses Molekül gemäß Anspruch 5, wobei die Aminosäuresequenz die Sequenz aus der Figur 5 hat.

7. Isoliertes Protein, das von einer Nukleinsäure kodiert wird, welche die Nukleinsäure von Position 2029 bis Position 3542 der Nukleinsäuresequenz, wie sie in Figur 5 dargestellt ist, umfasst.

8. Verwendung der Nukleinsäure gemäß Anspruch 1 oder 2, eines proteinösen Moleküls gemäß Anspruch 5 oder 6 oder eines Proteins gemäß -Anspruch 7 oder eines Antikörpers, der für ein proteinöses Molekül gemäß Anspruch 5 oder 6 spezifisch ist, für eine *in vitro* Diagnose von Mukosa-assoziierten Lymphoidgewebelymphomen.

9. Antikörper, der für ein Protein gemäß Anspruch 7 spezifisch ist.

## Revendications

1. Acide nucléique isolé et/ou recombinant codant pour une molécule protéique comprenant au moins les trois domaines BIR de l'inhibiteur d'apoptose 2 (API2) fusionnés au moins au domaine VDJ4 de la protéine de Translocation associée au lymphome de MALT (MLT) ayant la séquence des acides aminés 970-1013 de la figure 5.

2. Acide nucléique isolé et/ou recombinant selon la revendication 1 dans lequel la séquence d'acide nucléique a la séquence de la figure 5.

3. Véhicule d'administration nucléique comprenant un acide nucléique selon la revendication 1 ou 2.

4. Cellule comprenant un acide nucléique selon la revendication 1 ou 2.

5. Molécule protéique codée par un acide nucléique selon la revendication 1 ou 2.

6. Molécule protéique selon la revendication 5 dans laquelle la séquence d'acide aminé a la séquence de la figure 5.

7. Protéine isolée codée par un acide nucléique comprenant l'acide nucléique de la position 2029 à la position 3542 de la séquence d'acide nucléique décrite dans la figure 5.

8. Utilisation d'acide nucléique selon la revendication 1 ou 2, d'une molécule protéique selon la revendication 5 ou 6, ou d'une protéine selon la revendication 7 ou d'un anticorps spécifique pour une molécule protéique selon la revendication 5 ou 6 pour le diagnostic in vitro de lymphomes du système lymphoïde des muqueuses.

9. Anticorps spécifique d'une protéine selon la revendication 7.
